(19) Europäisches Patentamt
European Patent Office
Office européen des brevets

(11) Veröffentlichungsnummer: **0 000 880**
**B1**

(12) **EUROPÄISCHE PATENTSCHRIFT**

(21) Anmeldenummer: **78100571.5**

(22) Anmeldetag: **02.08.78**

(51) Int. Cl.³: **C 07 C 68/06**
C 07 C 69/96

(54) **Verfahren zur Herstellung aromatischer Kohlensäureester**

(30) Priorität: **10.08.77 DE 2736063**

(43) Veröffentlichungstag der Anmeldung:
**07.03.79 Patentblatt 79/05**

(45) Bekanntmachung des Hinweises auf die Patenterteilung:
**14.01.81 Patentblatt 81/02**

(84) Benannte Vertragsstaaten:
**DE FR GB NL**

(56) Entgegenhaltungen:
**DE - A - 2 528 412**
**FR - A - 2 291 967**

(73) Patentinhaber: **BAYER AG**
**Zentralbereich Patente, Marken und Lizenzen**
**D - 5090 Leverkusen 1, Bayerwerk (DE)**

(72) Erfinder: **Krimm, Heinrich, Dr.**
**Heyenbaumstrasse 65**
**D - 4150 Krefeld (DE)**
**Buysch, Hans-Josef, Dr.**
**Brandenburger Strasse 28**
**D - 4150 Krefeld (DE)**
**Rudolph, Hans, Dr.**
**Haydnstrasse 9**
**D - 4150 Krefeld (DE)**

**EP 0 000 880 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

# Verfahren zur Herstellung aromatischer Kohlensäureester

Die Erfindung betrifft ein Verfahren zur Herstellung aromatischer Kohlensäureester aus Dimethylcarbonat und Phenolen durch Umesterung.

Die Umesterung aliphatischer Kohlensäureester mit Phenolen in Anwesenheit starker Basen bzw. von Alkaliverbindungen ist nach DBP 971 790, 1 020 184, 1 026 958 und 1 031 512 bekannt. Derart katalysierte Umesterungsverfahren haben den Nachteil, wenig selektiv zu sein, da in einer Nebenreaktion erhebliche Mengen an Kohlendioxid freigesetzt werden.

In der DE—OS 2 528 412 ist ein entsprechendes Umesterungsverfahren zur Herstellung aromatischer Kohlensäureester in Gegenwart von Lewissäuren, d.h. Übergangsmetallhalogeniden, oder den entsprechenden Acyloxy-, Alkoxy- oder Aryloxyverbindungen als Katalysatoren beschrieben. Wird hierbei als Ausgangsmaterial Dimethylcarbonat verwendet, dann muß eine aufwendige Reinigungs- und Trennoperation zur Gewinnung reinen Dimethylcarbonats vorgenommen werden (USP 3 803 201, DBP 2 450 856 und DE—OS 2 607 003), da bei den bekannten Herstellungsverfahren von Dimethylcarbonat (USP 2 642 858 und DE—OS 2 615 665) ein etwa 30 Gew.-% Azeotrop mit Methanol anfällt. Gemäß DE—OS 2 528 412 wird somit Dimethylcarbonat mit Phenolen zu den entsprechenden aromatischen Carbonaten umgeestert, wobei Umesterungskatalysatoren vorhanden sind. Insbesondere wird im Beispiel 9 in Anwesenheit von n-Hexan gearbeitet und aus dem Gemisch von Dimethylcarbonat, Phenol, Hexan und Methylalkohol ein Azeotrop aus Hexan, Dimethylcarbonat und Methylalkohol abdestilliert. Dies hat zur Folge, daß ständig Hexan und Dimethylcarbonat nachgeliefert werden muß. Anders gesagt, mit dem Methylalkohol, der aus der Reaktion entfernt werden soll, wird auch Hexan und Dimethylcarbonat abgetrennt, was eigentlich unerwünscht ist.

Es stellte sich deshalb die Aufgabe, ein Umesterungsverfahren zu finden, das die Verwendung dieses Azeotrops bzw. eines sonstigen Gemisches aus Dimethylcarbonat und Methanol gestattet. Die Konzentration von Dimethylcarbonat in Methanol liegt im Bereich von 95 bis 10 Gew.-%. Bevorzugt wird das technisch wichtige 30 Gew.-% Azeotrop.

Die Lösung dieser Aufgabe gelang durch die Mitverwendung von mit Methanol nicht mischbaren Azeotropbildnern für Methanol.

Gegenstand der Erfindung ist daher ein Verfahren zur Herstellung aromatischer Kohlensäureester durch Umesterung von Dimethylcarbonat mit Phenolen unter Abspaltung von Methanol in Gegenwart von Umesterungskatalysatoren, das dadurch gekennzeichnet ist, daß man zur Umesterung Gemische aus Dimethylcarbonat/Methanol und mit Methanol nicht mischbare Azeotropbildner für Methanol verwendet.

Erfindungsgemaß geeignete Azeotropbildner sind vorzugsweise gesättigte aliphatische Kohlenwasserstoffe mit $C_5$—$C_8$ und mit Siedepunkten von 40 — 130°C, wie Pentan, Hexan, Heptan, Octan und Isooctan, ferner technische Benzinfraktionen, die vorwiegend die genannten Kohlenwasserstoffe enthalten, wie Petrolether, Ligroin und Leichtbenzin, sowie Gemische der genannten Kohlenwasserstoffe.

Die Azeotropbildner werden zweckmäßig zumindest in einer solchen Menge eingesetzt, die ausreicht, sowohl das mit dem Dimethylcarbonat/Methanol-Gemisch eingebrachte als auch das während der Umesterung gebildete Methanol überzudestillieren. Die zuzusetzende Menge an Azeotropbildner kann den bekannten Tabellen (z.B. in Handbook of Chemistry and Physics, 51. Edit. (1970), the Rubber Comp., Cleveland/Ohio) mit den Angaben über die jeweiligen Azeotropzusammensetzungen entnommen oder durch einfache Vorversuche ermittelt werden. Ein Überschuß an Azeotropbildner schadet nicht, er ist nur im Interesse einer ökonomischen Energiebilanz möglichst niedrig zu halten.

Überraschenderweise gelingt es mit dem erfindungsgemäßen Verfahren, das aus dem während der Umesterungsreaktion gebildeten Methanol und einem der genannten Kohlenwasserstoffe bestehende Azeotrop abzutrennen, obwohl diese Kohlenwasserstoffe bekanntlich auch mit Dimethylcarbonat azeotropsiedende Gemische bilden. Nach dem Verfahren der Erfindung werden praktisch die gleichen Ausbeuten wie nach dem bekannten Verfahren erzielt, ohne daß das eingesetzte Dimethylcarbonat vor der Umesterungsreaktion isoliert werden muß.

Als Phenole eignen sich vorzugsweise Verbindungen der allgemeinen Formel (I)

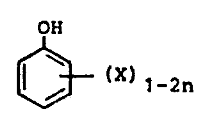

(I)

in der X für Wasserstoff, einen Alkylrest mit $C_1$—$C_3$, ein Halogenatom, vorzugsweise Chlor, oder eine Nitrogruppe und n für 1 oder 2 stehen, besonders bevorzugt werden Phenol, o,m,p-Kresol, o,m,p-Chlorphenol, o,m,p-Äthylphenol, o,m,p-Propylphenol, o,m,p-Nitrophenol, 2,6-Dimethylphenol, 2,4-Dimethylphenol oder 3,4-Dimethylphenol verwendet.

Anstelle der einwertigen Phenole können auch Bisphenole wie Dihydroxydiarylalkane mit $C_1$—$C_4$ im Alkylrest wie z.B. Bisphenol A, eingesetzt werden.

Als Katalysatoren können die bekannten Umesterungskatalysatoren verwendet werden.

Vorzugsweise sind dies Alkaliverbindungen wie Lithium-, Natrium-, Kaliumhydroxide, -alkoholate, -phenolate, -carboxylate und -carbonate. Vorzugsweise werden auch zinnorganische Verbindungen wie Trimethylzinnacetat, Triäthylzinnbenzoat, Tributylzinnacetat, Triphenylzinnacetat, Dibutylzinnacetat, Dibutylzinndilaurat, Dioctylzinndilaurat, Dibutylzinnadipinat, Methoxytributylzinn, Methoxytriphenylzinn, Phenoxytriäthylzinn, Dimethyldibutylzinn, Dimethylzinnglykolat, Diäthoxydibutylzinn, Diphenoxydibutylzinn, Dimethoxydiphenylzinn, Triäthylzinnhydroxid, Triphenylzinnhydroxid, Hexaäthylstannoxan, Hexabutylstannoxan, Tetrabutyldiphenoxystannoxan, Dibutylzinnoxid und Dioctylzinnoxid und Titanverbindungen vom Typ des Tetrabutyl- oder Tetraphenyltitanats eingesetzt.

Die Katalysatoren werden in Konzentrationen von etwa 0,001-20 Gew.%, bezogen auf die gesamte Reaktionsmenge, eingesetzt. Das Gewichtsverhältnis von Dimethylcarbonat: Phenol kann in weiten Grenzen schwanken und zwischen etwa 1:99 und 99:1, vorzugsweise 1:9 und 9:1 liegen. Von diesem Verhältnis hängt es ab, ob im Endproduckt Arylphenylcarbonat oder Diarylcarbonat überwiegt.

Ohne Schwierigkeit kann des neben Diarylcarbonat gebildete Methylarylcarbonat durch Destillation abgetrennt und entweder mit frischem Phenol umgesetzt oder nach Abtrennung des Diarylcarbonats zur weiteren Umsetzung zurückgeführt werden.

Die Reaktionstemperaturen liegen bevorzugt im Bereich von 50 — 250°C, besonders bevorzugt von 100 — 200°C. Vorteilhafterweise wird bei einem Druck von 1 Torr bis 20 Atms., vorzugsweise bei 1—5 Atms gearbeitet.

Die bevorzugte Verfahrensweise besteht darin, das Umesterungsgemisch an einer längeren Kolonne auf die gewünschte Reaktionstemperatur zu bringen, das Methanol in dem Maße, wie es im Reaktionsgut freigesetzt wird, zusammen mit dem Azeotropbildner gegebenenfalls mit Hilfe eines inerten Gasstromes abzutrennen und Dimethylcarbonat mit dem Azeotropbildner in dem Maße wie das Reaktionsgut an beiden Stoffen verarmt, dem unteren Teil der Kolonne zuzuführen.

Die Verfahrensproduckte können in bekannter Weise zu Polycarbonaten umgesetzt werden oder dienen als Ausgangsstoffe für Pflanzenschutzmittel.

Beispiel

a) An einer mit Glasringen beschickten 2,9 m hohen verspiegelten Füllkörperkolonne werden 940 g (10 Mol) Phenol, 150 g 30 proz. Dimethylcarbonat/Methanol-Azeotrop (0,5 Mol), 100 g n-Heptan und 2 g Tetrabutyltitanat erhitzt, so daß bei 58—59°C ein im wesentlichen aus Heptan und Methanol bestehendes zweiphasiges Azeotrop abgenommen werden kann. Im Laufe der 45 stdg. Reaktionszeit wird die dem abdestillierten Methanol/Heptan-Gemisch entsprechende Menge Dimethylcarbonat und Heptan in der Mitte der Kolonne so zugesetzt, daß die Sumpftemperatur 160°C beträgt. Insgesamt werden auf diese Weise 340 g Dimethylcarbonat/Methanol-Azeotrop mit 400 g Heptan entsprechend 1,13 Mol Dimethylcarbonat eingesetzt. Das Reakationsgut wird über eine 1 m hohe Kolonne fraktioniert. Nach einem aus Methanol, Dimethylcarbonat und Heptan bestehenden Vorlauf gehen bei 69 — 74°C/8 Torr 887 g nicht umgesetztes Phenol, bei 83 — 93°C/8 Torr 30,6 g Methylphenylcarbonat und bei 146 — 160°C/8 Torr 32.5 g Diphenylcarbonat über. Somit beträgt die Ausbeute an aromatischen Carbonaten, bezogen auf umgesetztes Phenol 94 % d. Th.

b) Ein Gemisch von 45,6 g (0,2 Mol) 2,2-Bis-(4-hydroxyphenyl)-propan, 47,1 g (0,22 Mol) des nach a) hergestellten Diphenylcarbonats und 0,008 g Natriummethylat wird langsam bis auf 210° unter 20 Torr erhitzt, wobei die Hauptmenge des abgespaltenen Phenols abdestilliert. Dann wird der Druck auf 0,2 Torr ermäßigt und die Temperatur während einer Stunde auf 250°C und während zweier weiterer Stunden auf 280°C erhöht, bis die Schmelze so zäh geworden ist, daß sie sich kaum mehr rühren läßt. Beim Abkühlen erhält man einen klaren, farblosen, elastischen Kunstoff, aus dessen Schmelze Formkörper mit hervorragenden Festigkeitseigenschaften hergestellt werden können.

## Patentansprüche

1. Verfahren zur Herstellung aromatischer Kohlensäureester durch Umesterung von Dimethylcarbonat mit Phenolen unter Abspaltung von Methanol in Gegenwart von Umesterungskatalysatoren, dadurch gekennzeichnet, daß man zur Umesterung Gemische aus Dimethylcarbonat/Methanol und Azeotropbildnern für Methanol, die mit Methanol nicht mischbar sind, verwendet und während der Umsetzung das Methanol/Azeotropbildner-Gemisch abdestilliert.

2. Verfahren nach Anspruch 1, dadurch gekennzeichnet, daß als Azeotropbildner gesättigte aliphatische Kohlenwasserstoffe mit $C_5$—$C_8$ oder technische Benzinfraktionen, die vorwiegend die genannten Kohlenwasserstoffe enthalten, verwendet.

3. Verfahren nach Ansprüchen 1—2, dadurch gekennzeichnet, daß die Konzentration des Dimethylcarbonats in Methanol im Bereich von 10 bis 95 Gew.% liegt.

4. Verfahren nach Ansprüchen 1—3, dadurch gekennzeichnet, daß die Umesterung bei Temperaturen von 50—250°C durchgeführt wird.

## Revendications

1. Procédé de production d'esters aromatiques d'acide carbonique par transestérification de carbonate de diméthyle avec des phénols, avec élimination de méthanol, en présence de catalyseurs de transestérification, caractérisé en ce qu'on utilise pour la transestérification des mélanges de carbonate de diméthyle et méthanol et d'agents formant un azéotrope pour le méthanol, qui ne sont pas miscibles au méthanol, et on chasse le mélange de méthanol et d'agent formant un azéotrope par distillation pendant la réaction.

2. Procédé selon la revendication 1, caractérisé en ce qu'on utilise comme agents formant un azéotrope des hydrocarbures aliphatiques saturés en $C_5$ à $C_8$ ou des fractions techniques d'essence qui contiennent principalement les hydrocarbures mentionnés.

3. Procédé suivant les revendications 1 et 2, caractérisé en ce que la concentration du carbonate de diméthyle dans le méthanol se situe dans la plage de 10 à 95% en poids.

4. Procédé suivant les revendications 1 à 3, caractérisé en ce que la transestérification est conduite à des températures de 50 à 250°C.

**Claims**

1. A process for the production of aromatic carbonic acid esters by transesterifying dimethyl carbonate with phenols under the elimination of methanol in the presence of transesterification catalysts, characterised in that for the transesterification mixtures of dimethyl carbonate/methanol and methanol-immiscible azeotrope formers for methanol are used and the mixture of methanol and azeotrope former is distilled off during the reaction.

2. A process according to claim 1, characterised in that the azeotrope formers used are saturated aliphatic $C_5$—$C_8$ hydrocarbons or commercial gasoline fractions which predominantly contain the above-mentioned hydrocarbons.

3.. A process according to claims 1 and 2, characterised in that the concentration of the dimethyl carbonate in methanol is within the range of 10 to 95% by weight.

4. A process according to claims 1 to 3, characterised in that the transesterification is carried out at temperatures from 50 to 250°C.